# EUROPEAN PATENT SPECIFICATION

(11) **EP 4 419 916 B1**
(45) Date of publication and mention of the grant of the patent: **01.04.2026**
(21) Application number: 22808978.5
(22) Date of filing: 18.10.2022
(51) Int. Cl.: G01N 33/558

(54) **TEST SYSTEM FOR OBTAINING A BLOOD COUNT SUITABLE FOR HOME USE AND TELEMEDICINE**
TESTSYSTEM ZUR ERSTELLUNG EINES BLUTBILDES FÜR DIE HEIMANWENDUNG UND EINE TELEMEDIZIN-BEHANDLUNG
SYSTEME DE TEST POUR L'OBTENTION D'UNE COMPTE SANGUIN ADAPTE A UN USAGE A DOMICILE ET A LA TELEMEDECINE

(30) Priority: 18.10.2021 DE 102021126982
(43) Date of publication of application: 28.08.2024
(73) Proprietor: Immundiagnostik AG, 64625 Bensheim (DE)
(72) Inventor: ARMBRUSTER, Franz Paul, 64625 Bensheim (DE); WALZER, Felix, 64625 Bensheim (DE); AL-ALI, Haifa Kathrin, 06120 Halle ( Saale) (DE); SCHULZE, Susann, 06120 Halle ( Saale) (DE)
(74) Representative: Benedum, Ulrich Max
(86) International application number: PCT/EP2022/078990
(87) International publication number: WO 2023/066942

(56) References cited:
- EP-A1- 0 905 514
- MITTAG ANJA ET AL: "Small and cheap: accurate differential blood count with minimal sample volume by laser scanning cytometry (LSC)", SPIE SMART STRUCTURES AND MATERIALS + NONDESTRUCTIVE EVALUATION AND HEALTH MONITORING, 2005, SAN DIEGO, CALIFORNIA, UNITED STATES, vol. 5692, 1 April 2005 (2005-04-01), US, pages 79 - 90, XP093022014, ISSN: 0277-786X, ISBN: 978-1-5106-4548-6, Retrieved from the Internet <URL:https://www.spiedigitallibrary.org/conference-proceedings-of-spie/5692/1/Small-and-cheap--accurate-differential-blood-count-with-minimal/10.1117/12.588653.full?SSO=1> DOI: 10.1117/12.588653
- NEUMULLER J. ET AL: "Demonstration by Flow Cytometry of the Numbers of Residual White Blood Cells and Platelets in Filtered Red Blood Cell Concentrates and Plasma Preparations", VOX SANGUINIS, vol. 73, no. 4, 1 November 1997 (1997-11-01), CH, pages 220 - 229, XP093021675, ISSN: 0042-9007, DOI: 10.1046/j.1423-0410.1997.7340220.x
- OYAERT M N ET AL: "Quantitative urine test strip reading for leukocyte esterase and hemoglobin peroxidase", vol. 56, no. 7, 27 June 2018 (2018-06-27), pages 1126 - 1132, XP009542366, ISSN: 1434-6621, Retrieved from the Internet <URL:http://www.degruyter.com/view/j/cclm.2018.56.issue-7/cclm-2017-1159/cclm-2017-1159.xml> DOI: 10.1515/CCLM-2017-1159
- SAITO SHUNNICHI ET AL: "Platelet-, leucocyte- and red cell-derived microparticles in stored whole blood, with and without leucofiltration, with and without ionising radiation", BLOOD TRANSFUSION = TRASFUSIONE DEL SANGUE, 1 February 2018 (2018-02-01), Italy, pages 145 - 153, XP093021673, Retrieved from the Internet <URL:https://www.ncbi.nlm.nih.gov/pmc/articles/PMC5839611/pdf/blt-16-145.pdf> [retrieved on 20230207], DOI: 10.2450/2016.0108-16

## Description

### FIELD OF INVENTION

The invention relates to a method for investigating the properties of particles, in particular the number of cells (leucocytes, erythrocytes, and thrombocytes) in whole blood, and to a device for measuring and testing the properties of blood in vitro, particularly in connection with a treatment of a recurrent neoplasm or leukemia (G01N33/574).

### TECHNICAL BACKGROUND

US 2016/0231225 A1 (Siemens Healthcare GmbH) discloses an *in vitro* method for determining a cell type of a cell in a blood sample comprising a microscopic device configured to detect a height profile of the cell to a carrier plate. The cell analysis device comprises determining cells according to the detected height profiles.

EP (Bioscientia GmbH) discloses a screening method for the diagnosis of hematological neoplasms in which a haematogram is produced with a blood sample based on the first set of filter rules, and if it deviates from the desired state, is checked for a potential hematological neoplasm using a computer program.

EP0905514 (Horiba Ltd) describes a compact combined blood cell counting and immunoassay device which comprises a station for withdrawing a whole blood sample and a nozzle member capable of selective taking whole blood from the blood sample. A blood cell counting section and an immunoassay section are mounted along a unitary path of movement of the nozzle member so that a single nozzle member can be used to transport the whole blood to the respective blood cell counting section and immunoassay section. A controller can automatically direct a transport mechanism to use only whole blood and to perform various steps of adding dilution liquid and reagents, as well as appropriate washing of the nozzle and sample cells, to ensure automatic operation. A hematocrit value can be determined from the blood cell count signal and used to correct the immunoassay signal.

US20210105979 A1 (Mars Inc.) describes a method for determining the biological age of an adult dog, the method comprising determining the values of blood globulin, total protein in the blood, alkaline phosphatase, platelet count, mean corpuscular volume in blood or urine specific gravity and comparing the results with values obtained from healthy dogs of a known age as well as kits, systems and/or computer media for performing the method.

US 20210039093 (Medica Corporation) describes single-use test cartridges, a cell analyzer apparatus, and methods for automatically performing microscopic cell analysis tasks, such as counting and analyzing blood cells in biological samples. A small, measured amount of whole blood is placed in a mixing dish on the disposable test cartridge after insertion into the cell analyzer. The analyzer also adds a known amount of diluent/stain in the mixing dish and mixes it with the blood. The analyzer takes a measured amount of the mixture and places it in a sample cup on the cartridge in fluid communication with an imaging chamber. The geometry of the imaging chamber is such that the uniformity of the mixture is maintained and the cells are not crowded or clumped together during transfer to the imaging chamber. The images of all the cellular components in the imaging chamber are counted and analyzed to obtain a complete blood count.

Further background art relating to blood cell count can be found in:
- Mittag et al. in Small and cheap: accurate differential blood count with minimal sample volume by laser scanning cytometry (LSC), SPIE SMART STRUCTURES AND MATERIALS + NONDESTRUCTIVE EVALUATION AND HEALTH MONITORING, 2005, 5692(1), 79-90;
- Neumueller et al., in Demonstration by Flow Cytometry of the Numbers of Residual White Blood Cells and Platelets in Filtered Red Blood Cell Concentrates and Plasma Preparations, VOX SANGUINIS. 1997, 220-229;
- Oyaert MN et al., in Quantitative urine test strip reading for leukocyte esterase and hemoglobin peroxidase, CLIN CHEM LAB MED, 2018, 56(7), 1126-1132;
- Saito S et al, Platelet-, leucocyte- and red cell-derived microparticles in stored whole blood, with and without leucofiltration, with and without ionising radiation, Blood transfusion = Transfusione del sangue, 2018, 145-153.

Home or personal testing can minimize the need for monitoring tests in clinics or laboratories, reduce healthcare costs, and improve people's quality of life. Testing remains an important aspect of quality of life for many people, especially the elderly and the infirm, who are unable or unwilling to travel to access appropriate monitoring services to maintain their health. Home testing is hampered by the complexity of medical instruments, the difficulty of use, the cost, and the lack of availability to the public. Several challenges exist in developing and deploying systems, devices, and methods that enable laboratory-quality testing outside of a laboratory.

Patients with haematologic neoplasms must have their blood counts checked regularly. Currently, these blood count checks are only performed in medical facilities where patients also have to wait for the results of the blood count check. This often takes a very long time. For elderly patients from sparsely populated, rural, or structurally weak areas, the often long journeys to the medical facility are also associated with considerable personal burdens and restrictions, very often also of a financial nature, and in many cases, the start of therapy is also delayed because the blood tests are then scheduled at longer intervals. For modern telemedicine, digital blood count monitoring is an unsolved medical problem.

### BRIEF DESCRIPTION OF THE INVENTION

The problem is addressed by a method of performing a blood cell count as disclosed in claims 1 to 10. The therein disclosed method enables the quantification of red blood cells (RBCs, erythrocytes), white blood cells (leucocytes), and platelet cells (thrombocytes) in a whole blood sample from an individual. The method comprises the steps:
a) providing a defined amount of a whole blood sample obtained from the said individual;
b) diluting and treating the defined amount of whole blood in cell lysis buffer for efficient cell lysis and solubilization of cell membrane proteins;
c) applying the resulting solution containing solubilized proteins and particulates from blood cells onto a sample pad of an immunochromatographic assay strip which comprises a membrane or fleece impermeable for particulates and permeable for solubilized proteins;
d) contacting the solubilized proteins of the whole blood sample with labeled receptors that bind to hemoglobin generally present in erythrocytes, or to CD42b antigen generally present on thrombocytes or to CD45 antigen generally present on leucocytes;
e) performing an immunochromatographic assay for each labeled receptor that has bound any one of the target analytes hemoglobin, CD42b, and CD45; and
f) quantifying the amounts of each of the target analytes CD45, CD42b, and hemoglobin; and
g) comparing and correlating the levels of CD45, CD42b, and hemoglobin to expected values and ranges found in healthy individuals and/or previously measured in the blood of an individual with a diagnosed neoplasm, where values outside the individually acceptable range or normal range indicate that the individual being studied will benefit from therapy.

In some preferred embodiments, a certain defined amount of whole blood is collected by capillary action. This may be done through a glass capillary tube of a diameter of 0,6 mm to 1.5 mm as commonly used for blood collection, but with a specific length and volume.

In some preferred embodiments, the capillary tube containing the specifically collected whole blood is placed into a vessel containing a predetermined amount of cell lysis buffer containing detergents for extracting and solubilizing single-pass transmembrane proteins to obtain a blood cell protein extract. The collected whole blood sample is diluted in the extraction buffer, the cells are lysed, and single-pass transmembrane proteins are extracted by the disruptive and chaotropic effects of the agents and detergents on the cell membranes.

In some preferred embodiments, a specific amount of recombinant fragment or isolated single-pass membrane protein of CD42b and/or CD45 is added to the extraction to allow quantification by competitive immunoassay.

In some preferred embodiments, the amount of blood cell extract is applied in equal aliquots to conjugate pads containing labeled anti-CD45 antibodies, labeled anti-immunochromatographic must contain successive binding zones containing labeled anti-CD45 antibodies, labeled anti-CD42b antibodies, and labeled anti-hemoglobin antibodies which may interact unfavorably in addition to the effects of the unavoidable binding zone for the control. A skilled person knows that the conjugate pads are then part of immunochromatographic test systems specific for CD45 antigen, CD42 antigen, and hemoglobin.

In some preferred embodiments, the blood cell extract is applied in equal aliquots on separate conjugate pads comprising labeled anti-CD45 antibodies, labeled anti-CD42b antibodies, and labeled anti-hemoglobin antibodies, respectively.

In some preferred embodiments, the conjugate pads contain defined amounts of labeled antibodies against an abundant human serum protein or commonly occurring protein as a control.

In some alternative embodiments, the antibodies to hemoglobin, CD45 antigen, CD42b antigen, and control protein are included in the cell lysis and extraction buffer or added to the cell lysis and extraction buffer or the running buffer of the specific immunochromatographic assay systems prior to their application onto the sample pads.

Another aspect of the invention relates to a kit of parts for performing a blood count as described above, comprising:
- a device for drawing and collecting a defined amount of whole blood;
- a vessel containing a predetermined amount of cell lysis and extraction buffer for dilution of the collected defined amount of blood, for lysing blood cells, for protein extraction, and solubilizing single-pass transmembrane proteins;
- one or more specific immunochromatographic test systems for CD45 antigen, CD42b antigen, and hemoglobin.

Another aspect of the method of the invention is a software for use with the specific immunochromatographic testing system and method for a small blood cell count as described and claimed in a smart phone comprising a digital camera, a light source, and a processor, the software supporting acquisition of digital images, reading of machine-readable representations of data, an exchange of data and image data with a remote server, and a display of information, data and test results on a display, wherein the software is configured to sequentially acquire and process a number of transient digital images - a video - to analyze each digital image (of the video) for the presence of a reference image, wherein the software is further configured to analyze each transient digital image of the video for the presence of a region of interest indicating the specific results of the immunochromatographic test systems, and if found, said software is configured to examine each region of interest for the measures of reflected light and discard any transient images where the measures of reflected light are inappropriate; and the software is configure to store image data from only suitable digital transient images for further analysis of the areas to select and average only images with suitable measures of light to determine by comparison the amount of haemoglobin, the number of leukocytes (white blood cells) and the number of thrombocytes (platelets) in the collected whole blood sample.

Further preferred aspects and embodiments of the disclosure will become apparent from the drawings, examples, detailed descriptions, and claims which defined the scope of the invention.

### BRIEF DESCRIPTION OF DRAWINGS

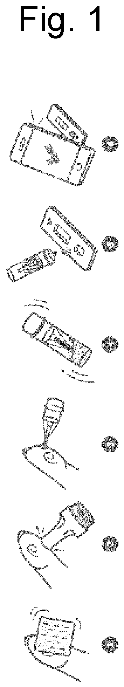

It is shown in: -
- **Fig. 1**: a schematic representation of the steps which must be performed by the patient to obtain and evaluate a blood count employing specific immunochromatographic test systems, the result of which is then transmitted by the smartphone app to the physician or medical facility for decision-making.

### DETAILED DESCRIPTION OF THE INVENTION

*Decision-making about treatment right before chemotherapy.* When chemotherapy is given for solid tumors, a small blood count must be done before every single chemo treatment to check that everything is in order. The blood count required for this, along with medical history and anamnesis, is usually responsible for about 80% of the delays before treatment. In addition, the blood count serves as a starting point for the therapy of solid tumors. Blood and mucosal cells, as rapidly dividing cell types, are particularly targeted by chemotherapy. Above all, the leukocyte and platelet values are decisive for the start of chemotherapy. A too-low Hb level can be counteracted by administering blood units. However, this always requires quantification of the patient's Hb level. If the Hb, leukocyte, and platelet values do not correspond to expected or normal ranges, the patient is sent home to recover and must present again after 2 to 3 days. This is always associated with enormous waiting times (approx. 3-4 hours on-site at the clinic) and travel times. If the patient could quantify these three parameters at home, he would be spared this very stressful procedure.

*Therapy monitoring.* If one wants to monitor the patient's condition during cancer treatment, a small blood count must be done about 1-2 times per month for solid tumors, and more frequently for hematological tumors. Up to now, this has mostly been done by the patient's family doctor. This doctor takes a blood sample from the patient and sends it to the laboratory. The result is then usually available about a day later. If there is a weekend in between, there can be a further delay of two to three days, even though in most cases the results are sent electronically or by telephone or e-mail. This procedure is disadvantageous because the blood count can be distorted by the transporting of the blood sample to the laboratory. In particular, the blood platelets (thrombocytes) can stick together so that the thrombocyte result is falsely low and a so-called pseudo-thrombocytopenia is diagnosed.

If blood values are poor in part to the side effects of the chemotherapy, among other things, the patient must present himself to the physician or medical facility as soon as possible to receive an infusion. Rapid initiation of treatment is critical and determines survival. Possible consequences of delayed initiation of therapy include infection, cerebral hemorrhage, or thrombosis. Statistical data show that the risks of not receiving timely treatment increase with factors such as poverty and distance to the primary care physician.

**TABLE 1**

| ***Measurement ranges and reference values*** | | | |
|---|---|---|---|
| Analyte | Measurement range | Normal range | Maximum expected concentration |
| Hemoglobin | < 3 gram/dl | 12 - 18g/dl | > 20g/dl |
| | -> 20 gram/dl | | |
| White Blood Cells | < 0.5 - > > 50 Gpt/L | 3,9 - 10,4 Gpt/L | >500 Gpt/L |
| Thrombocytes | < 10 -> > 400 Gpt/L | 140 - 360 Gpt/L | >3000 Gpt/L |

Use cases determine the measurement ranges.

The present disclosure encompasses diagnostic kits that contain a chromatographic specific binding assay system, and preferably an immunochromatographic specific binding assay system for hemoglobin, CD45 antigen as a quantitative pan-leukocyte marker, and CD42b surface protein as a quantitative marker of thrombocytes. CD stands commonly for a *cluster of differentiation* or *classification determinant* based on a protocol for the identification of cell surface molecules that allow immunophenotyping of cells.

The herein disclosed quantitative biomarker CD45 is known as protein tyrosine phosphatase, receptor type C (PTPRC), or leukocyte common antigen (LCA) and belongs to the protein tyrosine phosphatase family. The protein tyrosine phosphatases are signaling molecules that regulate a variety of cellular processes including cell growth, differentiation, mitotic cycle, and oncogenic transformation. CD45 is involved in the regulation of signal transduction in hematopoiesis. CD45 does not colocalize with human non-transformed hematopoietic cells, while CD45 positioning within lipid rafts is modified during their oncogenic transformation to leukemia. CD45 colocalizes with lipid rafts on AML cells, which contributes to elevated granulocyte-macrophage colony-stimulating factor (GM-CSF signal intensity) involved in the proliferation of leukemic cells. The granulocyte-macrophage colony-stimulating factor (GM-CSF) is an important hematopoietic growth factor and immune modulator and has profound effects on the functional activities of various circulating leukocytes.

CD45 is comprised of a 550 amino acid extracellular domain, a 20 amino acid transmembrane segment, and two large tandem intracytoplasmic catalytic domains. CD45 is a single-pass transmembrane protein and is present on all differentiated hematopoietic cells except erythrocytes and plasma cells. It can therefore be used as a biomarker for quantitating white blood cells (leukocytes) in a whole blood sample.

CD45 can be crystallized in hanging drops in 100 mM sodium acetate, pH 5.0, 25% glycerol, 10-13% PEG 4000, and 10 mM DTT with additives such as ammonium sulfate. Consequently, CD45 can easily be extracted from the membrane of white blood cells for quantitative analysis. The employed anti-CD45 antibodies bind to unique epitopes on the large extracellular domain which extends linearly out from the cell and bears the O-linked glycan chains. There are only eight isoforms of CD45 in the protein's large N-terminal region. The anti-CD45 antibodies against the CD45 isoforms are otherwise used in immunohistochemistry to differentiate between immune cell types and between histological sections from lymphomas and carcinomas. CD45 isoforms show cell-type and differentiation-stage specific expression, a pattern that is well conserved in mammals. The extracellular domain of CD45 is therefore a substitute biomarker for white blood cells (WBC) which encompass granulocytes (neutrophils, eosinophils, and basophils), monocytes, and lymphocytes (T cells and B cells). The amount of white blood cells or leukocytes in whole blood is part of a complete blood cell test used for the diagnosis of conditions such as infection, inflammation, allergy, and leukemia. It is preferred to use gold-labeled anti-CD45 antibodies that bind to all isoforms of the protein tyrosine phosphatase, receptor type C for quantitation of white blood cells in a drawn whole blood sample. The amount of CD45 antigen extractable from a whole blood sample can therefore be used to define a normal range as well as upper and lower cut-offs for leukocytes (white blood cells) in a blood sample.

CD42b antigen is also known as platelet glycoprotein Ib alpha polypeptide and in humans encoded by the GP1BA gene. The glycoprotein Ib (GP Ib) beta chain is also a single-pass membrane protein with a 120 amino acids long extracellular glycosylated N-terminal domain, an alpha-helical transmembrane portion of about 25 amino acids, and a 33 amino acid cytoplasmic domain. GP Ib (CD42) is soluble in 0.1 MMT buffer (DL-malic acid/MES/Tris, pH 5) and is likewise readily extractable from platelet membranes by adding Triton X-100 and 100 mM imidazole. The CD42b can associate with platelet glycoprotein IX and platelet glycoprotein V to form a glycoprotein Ib-IX-V complex. Several polymorphisms and mutations have been described in the GP1 BA gene, some of which are the cause of von Willebrand's disease. Notwithstanding, a quantitation of CD42b can be used as a substitute marker for a platelet count test (counting of thrombocytes) in a whole blood sample.

The platelet count test is generally used to monitor and diagnose conditions that cause too much bleeding or too much clotting and is typically included in a complete blood count. A lower than normal platelet count (thrombocytopenia) may indicate (return of) cancer that affects the blood (leukemia or lymphoma), a viral infection, such as mononucleosis, hepatitis, or measles, an autoimmune disease, infection or damage to the bone marrow, cirrhosis, vitamin B12 deficiency. A higher than normal platelet count (thrombocytosis), may also indicate certain types of cancer, such as lung cancer or breast cancer, anemia, inflammatory bowel disease, rheumatoid arthritis, or a viral or bacterial infection. Symptoms of thrombocytopenia include prolonged bleeding (nose bleeding, unexplained bruising, bleedings under the skin, etc. Symptoms of too many platelets include numbness of hands and feet, headache, dizziness, and weakness. The amount of CD42b antigen extractable from a whole blood sample can therefore be used to define a normal range as well as upper and lower cut-offs for thrombocytes (platelets) in a blood sample.

The amount of hemoglobin in a whole blood sample can be used as a substitute for the number of erythrocytes in a sample or the hematocrit(HT or HCT). which stands for the volume percentage (vol%) of red blood cells (RBC) in blood, measured as part of a blood test. An estimated hematocrit as a percentage may be derived by tripling the hemoglobin concentration in g/dL and dropping the units. In other words, the hematocrit is usually about 3 times the hemoglobin value (assuming there is no marked hypochromia). Reference values are 42-52% for males and 36-48% for females. It is a part of a person's complete blood count results, along with WBC count and platelet count. Because the purpose of red blood cells is to transfer oxygen from the lungs to body tissues, a blood sample's hematocrit is a reference to its capability of delivering oxygen. Haematocrit levels that are too high or too low can indicate a blood disorder, dehydration, or other serious medical conditions.

The system and apparatus are similar to systems for analyzing and quantifying the result of lateral flow immunochromatography (WO2019/215199A1, WO2021/180442A1; WO2020/165456A1). Lateral flow immunochromatography is suitable for use in the field, e.g. at home or a point of care, or in a physician's office, due to its accuracy and simple procedure steps. Test results can be read out using a smartphone with a camera so that the result is available within a few minutes and can be simultaneously transmitted to the attending physician via telephone or the Internet. Such systems are available and have already proven themselves in practice, also in the field of critical diagnostics important to live (cf., WO2019/215199A1, WO2021/180442A1; WO2020/165456A1). The combination of lateral flow immunochromatography and smartphone-based readout of the test result is a straightforward and safe entry into telemedicine. The combination is safe because both the immunochromatography device and the smartphone APP can be coded and certified so that their regular use can be monitored by the attending physician, and the smartphone-based readings transmitted to the physician can be immediately monitored and documented. Implausibilities in the readings can be detected and flagged by Al. Also, the patient does not have to be confronted with the specific result of the measurement, but the result of the reading shown can be: - 1) *The measurement was successful. Your blood values are unchanged; (2) The measurement was successful. Please contact your physician.* Or any other useful and accepted message. Therefore, the present disclosure is safe, easy to use, and requires only a minimum of skill and commitment from the user.

In one embodiment of the disclosure, a chromatographic-specific binding assay strip device is disclosed, comprising: a non-permeable platform strip, a permeable membrane testing strip positioned on top of the non-permeable platform strip, a sample receiving pad positioned on top of and at a proximal end of the non-permeable platform strip while in contact with the proximal end of the permeable membrane testing strip, a reservoir pad positioned on top of and at a distal end of the non-permeable membrane testing strip while in contact with the distal end of the permeable membrane test strip; a conjugate pad membrane attached to and extending from the proximal end of the non-permeable platform strip, and a conjugate pad positioned on said conjugate pad membrane, said conjugate pad comprising a permeable membrane containing a colorant conjugate. The permeable membrane is preferably impermeable to particulates. The colorant conjugate may be a gold-marked monoclonal antibody of hemoglobin, CD45, and CD42b.

The receptors may be one or more monoclonal antibodies against hemoglobin, CD45, and CD42b. In some embodiments, the monoclonal antibody may be a receptor that consists of the complement determining region (CDR) or an antigen-binding fragment (Fab), or a single-chain variable fragment (scFv) of a single-chain antibody against CD45 antigen, CD42b antigen, or against hemoglobin antigen fused with an amino acid chain from a protein or peptide not bound by molecules related to the immune system.

In some embodiments, the method is preferably part of a lateral flow immunoassay comprising an scFv receptor as described which is fused to an immunologically neutral blood protein, preferably HAS or haptoglobin, and coupled with gold particles as a label. In an alternative embodiment, the scFv receptor fused to an immunologically neutral protein is immobilized to a zone on a membrane of a lateral flow immunochromatography.

Using the above-described assay strip, a lysed whole blood sample or diluent thereof is prepared and poured into a test tube. Next, the conjugate pad of the lateral flow assay, preferably containing conjugated colloidal gold, is inserted into the sample in the test tube. It is most preferable to gently shake the tube, to encourage a complete mixture of all of the samples and all of the conjugate gold. After about five to twenty seconds or any fair amount of time to allow the ligand in the sample the conjugate gold to mix and bind, the assay strip is further pushed into the test tube so that the sample receiving pad is in contact with the colloidal gold-coated ligand sample. Capillary action will then draw the gold-coated ligand sample along the path of the lateral flow assay, and a positive result will show up at a line at the site of the appropriate anti-ligand antibody. The lateral flow assay can be withdrawn from the sample liquid after 5-15 seconds and placed in a horizontal position, or the lateral flow assay can remain in the sample liquid during the time that the sample is being drawn up by capillary action.

In some circumstances, particularly when a plastic cassette case is used to hold the lateral flow assay strip, it may be preferable to have the conjugate pad on the underside of the conjugate membrane. In another embodiment of the disclosure, a chromatographic-specific binding assay strip device is disclosed, comprising: a non-permeable platform strip, a permeable membrane testing strip positioned on top of the non-permeable platform strip, a sample receiving pad positioned on top of and at a proximal end of the non-permeable platform strip while in contact with the permeable membrane testing strip, a reservoir pad positioned on top of and at a distal end of the non-permeable membrane testing strip while in contact with the permeable membrane test strip; a semi-permeable membrane positioned on top of said sample pad; and a conjugate pad positioned on top of a said semi-permeable membrane, said conjugate pad comprising a permeable membrane containing a colorant conjugate.

A semi-permeable membrane, is a membrane that will allow certain molecules or ions to pass through it by diffusion and occasionally specialized "facilitated diffusion". The rate of passage depends on the pressure, concentration, and temperature of the molecules or solutes on either side, as well as the permeability of the membrane to each solute. Depending on the membrane and the solute, permeability may depend on solute size, solubility, properties, or chemistry. An example of a semi-permeable membrane is the lipid bilayer, the plasma membrane that surrounds all biological cells. Many natural and synthetic materials thicker than a membrane are also semi-permeable. An example of this is the thin film on the inside of an egg.

Another example of a semi-permeable membrane is a phospholipid bilayer, a group of phospholipids (consisting of a phosphate head and 2 fatty-acid tails) arranged into a double-layer, with the hydrophilic phosphate heads exposed to the water content outside and within the cell and the hydrophobic fatty-acid tails hidden in the inside. The phospholipid bilayer is the most permeable to small, uncharged solutes. Protein channels float through the phospholipids, and collectively, this model is known as the fluid mosaic model.

In the process of reverse osmosis, thin film composite membranes (TFC or TFM) are used. Thin film composite membranes are semi-permeable membranes manufactured principally for use in water purification or desalination systems.

The "cell lysis buffer" and extraction buffer for single-pass transmembrane antigens can be a buffer solution used for breaking open cells. A typical lysis buffer contains salts (e.g. Tris-HCI or EDTA) to regulate the acidity and osmolarity of the lysate. Detergents such as Triton X-100 or SDS may be added to break up membrane structures. In one embodiment the cell lysis buffer is a red blood cell lysis buffer, the typical components of which are ammonium chloride (NH₄Cl), sodium bicarbonate, EDTA, and water. In one nonlimiting example, CD45 cells (leucocytes and WBCs), CD42b cells (platelets, thrombocytes), and RBCs are the undesired cell types that are broken and lysed by the cell lysis buffer of the invention.

In some embodiments, the cell lysis buffer and membrane protein extraction buffer are a red blood cell lysis buffer comprising NH₄Cl and Na₂-EDTA, preferably an aqueous solution comprising 1-20 g/L NH₄Cl and 0.05-0.2 mM Na₂-EDTA, more preferably an aqueous solution comprising 5-10 g/L NH₄Cl and 0.1 mM Na₂-EDTA, pH 7.2-7.4. Before use, the red blood cell lysis buffer is filtered through a 0.22 µm microfiltration membrane and equilibrated to room temperature.

In some embodiments, the cell lysis and membrane protein extraction buffer may further comprise in molar excess relative to proteins and amino acids, preferably 1 to 50 mM/L of a ketocarboxylic acid having 1 to 12 carbon atoms and, optionally detergents, solubilizing agents, complexing agents, redox agents, biocides, adjuvants, and salts for the adjustment of ionic strength. The ketocarboxylic acid may be selected from α-keto monocarboxylic acids, α-keto dicarboxylic acids, β-keto carboxylic acids, β-keto dicarboxylic acids, α-keto propionic acid (pyruvic acid), acetoacetic acid, ketomalonic acid (mesoxalic acid), oxaloacetic acid, oxalic succinic acid, α-keto glutaric acid, isocitrate, oxohexanoic acid (ketocaproic acid), oxoheptanoic acid (ketoenanthic acid), Y-keto carboxylic acids, levulinic acid, phenylpyruvate acid, ketone derivatives and esters of terephthalic acid, and mono- or diesters of the above-mentioned keto carboxylic acids with a linear or branched alcohol having 1 to 6 carbon atoms, glyoxylic acid, glyoxylic acid ethyl ester, pyruvic acid ethyl ester. The detergent may be Triton^{®} X-100 in an amount from 0.001 to 5.0% by weight. In some embodiments, one or more zwitterionic detergent may be added, e.g. lauryl dimethylamine-N-oxide (LDAO), 3-[(3-Cocamidopropyl)dimethylammonio]-1-propane sulfonate (CHAPS), 3-[(3-cholamido-propyl)-dimethylammonio]-2-hydroxy-1-propane sulfonate (CHAPS), and alkyl maltosides and glycosides. Most preferable detergent for a solubilization of CD45 and CD42b are n-dodecyl-β-D-maltopyranoside (DDM), n-decyl-β-d-maltopyranoside (DM) and a mixture of n-octyl-β-d-glucopyranoside / n-nonyl-β-d-glucopyranoside (OG/NG). Other potential detergents for the cell lysis buffer and membrane protein extraction are n-undecyl-β-d-maltopyranoside (UDM), lauryl maltose neopentyl glycol (LMNG), Triton X-100, Thesit^{®}, Digitonin, Saponin, Cymal-5 and Cymal-6, and CHAPS. The cell lysis and membrane protein extraction solution may further contain, based on the total weight, preferably 0.005 to 0.5% by weight, of sugar and/or an amino sugar, preferably a preservative mono-, di- or trisaccharide, preferably sucrose, lactose, maltose, or trehalose. The amino sugar may be glucosamine, N-methyl glucosamine, galactosamine or neuraminic acid. Sugars such as sucrose or dextrose are not excluded. Trehalose may be added because of its properties as a "water substitute" in the stabilization of membrane proteins. The extraction buffer may contain a superplasticizer selected from polycarboxylate, polycarboxylate ether, naphthalene sulfonate, melamine sulfonate, and lignin sulfonate.

Ideally, the quantitative assay for the individual analytes (CD45, CD4b, and hemoglobin) is the specified measuring range. Cut-offs must be set for the upper limits, i.e. if values are higher than the upper limit, greater than X is displayed by the smartphone. Moreover, cut-offs must also be defined for the lower limits in the test system, i.e. if values are below the lower limit, a smaller Y is displayed by the smartphone. Preferably clear use cases must be defined for defined the given patient groups:- Solid oncology: Monitoring of treatment and relapse; Aftercare (= follow-up) for 5 years, Therapy management, control and monitoring (possibility of remote care); Decision on treatment strategy.

The patient-related outcome is important in clinical studies. Consequently, there is a need to examine the stated parameter in small studies and collect the corresponding data. An increased Quality of Life is therefore demonstrable by significantly reduced waiting times.

The rapid blood count test may be developed for therapy decision making which requires a smartphone app for a small measurement range. For follow-up of patients with chronic leukemia who need lifelong, ambulant permanent therapy (prevalence approx. 1 patient per 100.000 inhabitants per year), blood values can occur that are very high so larger measurement ranges would be required. The smartphone app and platform may also comprise implemented questionnaires and scores and the data may also be used for monitoring and follow-up, as well as The app should be linked to a CHESS trial, say a protocol for the process evaluation of a randomized trial of an education and self-management intervention for people. Patient-related outcomes are already collected using the CHESS (Chronic Headache Education and Self-management Study) database system, but the result of the smartphone app may give an additional focus on test results. CHESS works with a traffic light system which may be also used by the smartphone app for the patient. The smartphone portal is preferably patient-centered so that the patient determines who receives his data. Progress monitoring is a preferred embodiment as well as a link to teleconsultations or video telephony via smartphone.

The total number of granulocytes is a desirable 4th parameter. Granulocytes circulate through the body via peripheral blood and account for approximately 25-75% of CD45-positive leukocytes. However, there are several pan-granulocyte cellular markers such as CD32, CD33, CD15, CD13, or CD11b which multiply technical problems and complicate test evaluation.

### EXAMPLES

### EXAMPLE 1 - Test analysis

Immunochromatographic tests for CD45, CD42b, and hemoglobin are performed as shown in Figure 1 using a test device bearing reference images. Upon the appearance of visible signals (T, C bands), the hand-held smartphone is directed to the test cassette and a software application for image capturing is run using the digital camera. It is first searched for a bar code or QR whether the test is of an allowed production lot. When the reference image is found, the distance between the smartphone and the immunochromatographic test is assessed based on reference images on the cassette. This is preferably done on basis of the boundaries of the "viewport". Then the brightness and brightness gradient within the viewport is determined whether it contained any untypical dark areas (shadows). If the brightness within the viewport proves acceptable on the transient image, a section of the viewport is excised from the transient image, retrieved, and saved. Simultaneously, it is indicated to the user by a noticeable click that an image has been captured. Then any misalignment or skew of the saved image is corrected on basis of the control line (C) and the boundaries of the viewport. The saved and processed image is further processed for a determination of the peak intensities of the control and test lines (T, C) and then their ratio is determined.

More precisely, the transient image is evaluated using multiple metrics, namely brightness gradient, brightness, shadow, and facultatively, reflection and sharpness. When a metric is found outside the normal range, an error value is assigned, and the transient image will not be used. When the image matches the requirements, the relevant portion of the image is cut out and normalized, noise reduced, transformed into a relative "grayscale" (mean of the RGB), and, finally, the concentration of the analyte, namely CD45, CD42 and hemoglobin determined based on the intensity ratio of the signal zones (T, C). When there is an error on the transient image, this is not communicated to the user but simply the transient image discarded. After a definite number of total errors, the user obtains support based on the rated images. This helps to draw conclusions about the environment and the test itself. Solutions to avoid these errors are communicated to the user.

Transient Images are constantly taken (about 4 frames per second) and each transient image is analyzed continuously in the image stream until several evaluable transient images have been retrieved and saved, that is, displaying acceptable light reflection and brightness metrics (outside predetermined error value ranges). This helps to discover and avoid manipulations by the user. Commonly, seven acceptable and evaluable transient images are retrieved, the relevant portions cut out and analyzed as described, from which one result corresponding to the median value is chosen and the result is analyzed and reported.

### Example 2 - Blood count

Markers such as hemoglobin, CD45 antigen, and CD42b antigen can be used for carrying out a "small blood cell count" utilizing lateral flow immunoassay and for monitoring a neoplastic disease. The CD42b and CD45 antigens in lysed whole blood are pan-markers for white blood cells and platelets in a sample and their measurements are suited for assessing the disease activity.

The testing system can be made up of a modified test device (*Preventis^{®},* Bensheim, DE) and a software package. The test device was originally for a quantitative determination of vitamin D in the blood. This required that the calibration curve for the assessment of the bands is saved on a server that is accessible via software registration and a machine-readable production lot code. Similar can be done for CD45 antigen, CD42b antigen, and hemoglobin. In brief, a test device is provided consisting of immunological rapid tests for quantitation of human hemoglobin, CD45 antigen, and CD42b antigen via gold-conjugated anti-antigen antibodies. The system further comprises a pre-determined amount of non-analyte-specific antibodies in the conjugation pad to achieve a standard intensity of the control zone (C) independently from the amount of target antigen in the extracted blood sample. The result of the rapid test can be quantitatively assessed based on the ratio of the peak intensities of the lines for control (C) and tested analyte (T). Transient images are then retrieved, saved, processed, and assessed using the deposited lot-specific calibration data. An additional reference image (QR code) may be provided for authentication and confidentiality of data so that the respective smartphone is preferably first registered at the server, e.g. together with a code handed out by the supervising physician. The date, time, and result (median) can then be automatically sent to the patient's supervising physician.

In brief, the packaging comprised the following other items: a gauze piece for disinfection, a lance, preferably an automated lancet, a calibrated capillary blood collection tube, a vessel with a predetermined amount of cell lysis and extraction buffer, a specific immunochromatographic test cassette with strips for CD45, CD42b, and hemoglobin in the sealed aluminum pouch and a leaflet with instructions. The test cassette is removed from the aluminum pouch and placed on a flat, dry surface. A blood sample is drawn and the blood capillary is inserted into the buffer for cell lysis and extraction and solubilization of membrane proteins. The extraction tube is shaken gently for a few seconds and the bottom tip is broken off. Drops of extraction buffer with the extracted sample are applied onto the sample application window (conjugate pads) on the test cassette by evenly squeezing the sample collection tube, and then immediately pressing "start timer" in the *software* app. After 15 minutes the lateral flow chromatography is complete and can be analyzed.

For taking an image, the test cassette is placed on a bright, flat, and smooth surface. Shadows, strong light from the side, and direct sunlight should be avoided. A template of the test cassette is shown on the screen of the mobile phone. The test device is aligned in front of the user with the template on the screen. The smartphone app automatically triggers the camera which is a video screen (four frames per second) until seven suitable transient images with correct brightness in the viewport region and distance are found. Once the image taking was complete, the saved images are analyzed, and the median result is used for reporting and result presentation. The result is also sent to the supervising physician in a pseudo-anonymized form to protect personal data. Consequently, the patient is enabled to determine and record the blood count independently from the physician and the supervising medical center. On the other hand, the supervising physician receives the data for a prompt stepping up of the therapy in case of a disease flare. The disclosure relates to a system comprising a lateral flow test for use with a smartphone and allows patients and physicians to evaluate objectively the result of lateral flow chromatography. The system may therefore provide considerable time savings to patients.

The above examples and preferred embodiments are provided for clarification. The claimed scope of protection and the general idea of the invention is to be taken from the following patent claims.

## Claims

1. A method of quantifying red blood cells (erythrocytes), white blood cells (leucocytes), and platelet cells (thrombocytes) in a whole blood sample of an individual, the method comprising: -
a. providing a defined amount of a whole blood sample obtained from said, individual;
b. diluting and treating the defined amount of whole blood in cell lysis buffer for efficient cell lysis and solubilization of cell membrane proteins;
c. applying the resulting solution containing solubilized proteins and particulates from blood cells onto a sample pad of an immunochromatographic assay strip which comprises a membrane or fleece impermeable for particulates and permeable for solubilized proteins;
d. contacting the solubilized proteins of the whole blood sample with labeled receptors binding to hemoglobin that is generally present in erythrocytes, or CD42b antigen that is generally present on thrombocytes, or CD45 antigen that is generally present on leucocytes;
e. performing an immunochromatographic assay for each labeled receptor that has bound one of the target analytes hemoglobin, CD42b, and CD45; and
f. quantitation of the amounts of each target analyte CD45, CD42b, and hemoglobin; and
g. comparing and correlating the levels of CD45, CD42b, and hemoglobin with expected values and ranges found in healthy individuals and/or previously measured in the blood of an individual with a diagnosed neoplasia, with values outside the individual allowed range or a normal range indicating that the individual under study will benefit from therapy.

2. Method according to claim 1, wherein a defined amount of whole blood is collected by capillary action and extracted using a predetermined amount of cell lysis buffer comprising detergents for extraction and solubilization of single-pass transmembrane proteins to obtain a blood cell extract.

3. Method according to claim 2, wherein a defined amount of recombinant fragment of CD42b and/or CD45 or isolated single-pass membrane protein is added to the blood cell extract to allow for quantification by competitive immunoassay.

4. Method according to any claim 1 to 3, wherein the blood cell extract is applied in equal aliquots on separate conjugate pads comprising labeled anti-CD45 antibodies, labeled anti-CD42b antibodies, and labeled anti-hemoglobin antibodies, respectively.

5. Method according to any claim 1 to 3, wherein the blood cell extract is applied onto a single conjugate pad wherein the immunochromatographic test system has binding zones for CD45 antigen, CD42 antigen, and hemoglobin.

6. Method according to any preceding claims 1 to 5, wherein one or more conjugate pads contain defined amounts of labeled antibodies against an abundant human serum protein or commonly occurring blood protein as a control.

7. Method according to any claim 1 to 3, wherein antibodies against any one of hemoglobin, CD45 antigen, CD42b antigen, and control protein are contained in the cell lysis and extraction buffer or added directly to the cell lysis and extraction buffer.

8. A method of performing a blood cell count comprising quantifying red blood cells (erythrocytes), white blood cells (leucocytes), and platelets (thrombocytes) in a whole blood sample collected using an immunological assay comprising: -
a) providing a defined amount of a whole blood sample collected from an individual or person under chemotherapy or suffering from a solid or hematologic tumor;
b) diluting and treating the defined amount of whole blood in cell lysis buffer for efficient cell lysis and solubilization of cell membrane proteins;
c) applying the resulting solution containing solubilized proteins and particles from blood cells onto a sample pad of an immunochromatographic assay strip comprising a membrane or fleece or non-woven fabric impermeable to particles and permeable to solubilized proteins;
d) contacting the solubilized proteins of the whole blood sample with labeled receptors binding to hemoglobin generally present in erythrocytes, or CD42b antigen generally present on thrombocytes or CD45 antigen generally present on leucocytes;
e) performing an immunochromatographic assay for each labeled receptor that has bound any of the target analytes hemoglobin, CD42b, and CD45; and
f) quantifying the amounts of each of the target analytes CD45, CD42b, and hemoglobin; and
g) comparing and correlating the amounts of CD45, CD42b, and hemoglobin to expected values and ranges found in healthy individuals and/or previously measured, where values outside the individually acceptable range or normal healthy range indicate that the individual being studied will benefit from therapy.

9. A method of performing a blood cell count comprising quantifying red blood cells (erythrocytes), white blood cells (leucocytes), and platelets (thrombocytes) in a whole blood sample collected using an immunological assay as claimed in any claim 1 to 8, further comprising the used of a kit of parts for performing a blood count as claimed in any claim 1 to 8, comprising:
- a device for collecting a defined amount of whole blood by capillary action;
- vessel comprising cell lysis and extraction buffer for dilution of the drawn defined amount of blood, lysis of blood cells, protein extraction, and solubilization of single-pass transmembrane proteins;
- one or more specific immunochromatographic test systems for CD45 antigen, CD42b antigen, and hemoglobin.

10. A method of performing a blood cell count comprising quantifying red blood cells (erythrocytes), white blood cells (leucocytes), and platelets (thrombocytes) in a whole blood sample collected using an immunological assay as claimed in any claim 1 to 8 and a kit of parts as described in claim 9, further comprising the use of a software for use with a specific immunochromatographic testing system and method as claimed in any claim 1 to 9 and in a mobile phone comprising a digital camera, a source of light and a processor, which software supports the taking of digital images, the reading of machine-readable representations of data, an exchange of data and image data with a remote server, and a representation of information, data and test results on a display, wherein the software is configured to process sequentially several transient digital images and analyze each digital image for the presence of a reference image, and to analyse each transient digital image for a region of interest displaying the specific results of the immunochromatographic test systems, and if found, is configured to examine each region of interest for the measures of light reflected; and is configured to save the image data of said transient digital image for further analysis of the regions of interest to determine by comparison the amount of hemoglobin, the number of leukocytes (white blood cells), and the number of thrombocytes (platelets) in the whole blood sample drawn.

## Patentansprüche

1. Verfahren zur Quantifizierung von roten Blutkörperchen (Erythrozyten), weißen Blutkörperchen (Leukozyten) und Blutplättchen (Thrombozyten) in einer Vollblutprobe einer Person, wobei das Verfahren folgende Merkmale umfasst: -
a. Bereitstellen einer definierten Menge einer Vollblutprobe, die von der Person gewonnen wurde;
b. Verdünnen und Behandeln der definierten Menge Vollblut in einem Zelllysepuffer zur effizienten Zelllyse und Solubilisierung von Zellmembranproteinen;
c. Aufbringen der resultierenden Lösung, die solubilisierte Proteine und Partikel aus Blutzellen enthält, auf ein Probenkissen eines immunchromatographischen Teststreifens, der eine Membran oder ein Vlies umfasst, das für Partikel undurchlässig und für solubilisierte Proteine durchlässig ist;
d. Inkontaktbringen der solubilisierten Proteine der Vollblutprobe mit markierten Rezeptoren, die an Hämoglobin binden, das im Allgemeinen in Erythrozyten vorhanden ist, oder mit CD42b-Antigen, das im Allgemeinen auf Thrombozyten vorhanden ist, oder mit CD45-Antigen, das im Allgemeinen auf Leukozyten vorhanden ist;
e. Durchführen eines immunchromatographischen Assays für jeden markierten Rezeptor, der eines der Zielanalyten Hämoglobin, CD42b und CD45 gebunden hat; und
f. Quantifizierung der Mengen jedes Zielanalyten CD45, CD42b und Hämoglobin; und
g. Vergleichen und Korrelieren der Konzentrationen von CD45, CD42b und Hämoglobin mit den erwarteten Werten und Bereichen, die bei gesunden Personen und/oder zuvor im Blut einer Person mit diagnostizierter Neoplasie gemessen wurden, wobei Werte außerhalb des für die Person zulässigen Bereichs oder eines normalen Bereichs darauf hinweisen, dass die untersuchte Person von einer Therapie profitieren wird.

2. Verfahren nach Anspruch 1, wobei eine definierte Menge Vollblut durch Kapillarwirkung gesammelt und unter Verwendung einer vorbestimmten Menge eines Zelllysepuffers, der Detergenzien zur Extraktion und Solubilisierung von Single-Pass-Transmembranproteinen umfasst, extrahiert wird, um einen Blutzellextrakt zu erhalten.

3. Verfahren nach Anspruch 2, wobei eine definierte Menge eines rekombinanten Fragments von CD42b und/oder CD45 oder isoliertem Single-Pass-Membranprotein zu dem Blutzellextrakt hinzugefügt wird, um eine Quantifizierung durch einen kompetitiven Immunoassay zu ermöglichen.

4. Verfahren nach einem der Ansprüche 1 bis 3, wobei der Blutzellextrakt in gleichen Aliquoten auf separate Konjugatpads aufgetragen wird, die jeweils markierte Anti-CD45-Antikörper, markierte Anti-CD42b-Antikörper und markierte Anti-Hämoglobin-Antikörper umfassen.

5. Verfahren nach einem der Ansprüche 1 bis 3, wobei der Blutzellextrakt auf ein einzelnes Konjugatpad aufgebracht wird, wobei das immunchromatographischen Testsystem Bindungszonen für CD45-Antigen, CD42-Antigen und Hämoglobin aufweist.

6. Verfahren nach einem der vorstehenden Ansprüche 1 bis 5, wobei ein oder mehrere Konjugatpads definierte Mengen an markierten Antikörpern gegen ein reichlich vorhandenes Human-Serumprotein oder ein häufig vorkommendes Blutprotein als Kontrolle enthalten.

7. Verfahren nach einem der Ansprüche 1 bis 3, wobei Antikörper gegen eines von Hämoglobin, CD45-Antigen, CD42b-Antigen und Kontrollprotein in dem Zelllyse- und Extraktionspuffer enthalten sind oder direkt zu dem Zelllyse- und Extraktionspuffer hinzugefügt werden.

8. Verfahren zur Durchführung einer Blutkörperchenzählung, umfassend die Quantifizierung von roten Blutkörperchen (Erythrozyten), weißen Blutkörperchen (Leukozyten) und Blutplättchen (Thrombozyten) in einer Vollblutprobe, die unter Verwendung eines immunologischen Assays entnommen wurde, umfassend: -
a) Bereitstellen einer definierten Menge einer Vollblutprobe, die von einer Person entnommen wurde, die sich einer Chemotherapie unterzieht oder an einem soliden oder hämatologischen Tumor leidet;
b) Verdünnen und Behandeln der definierten Menge an Vollblut in einem Zelllysepuffer zur effizienten Zelllyse und Solubilisierung von Zellmembranproteinen;
c) Aufbringen der resultierenden Lösung, die solubilisierte Proteine und Partikel aus Blutzellen enthält, auf ein Probenkissen eines immunchromatographischen Teststreifens, der eine Membran oder ein Vlies oder ein nicht gewebtes Gewebe umfasst, das für Partikel undurchlässig und für solubilisierte Proteine durchlässig ist;
d) Inkontaktbringen der solubilisierten Proteine der Vollblutprobe mit markierten Rezeptoren, die an Hämoglobin binden, das im Allgemeinen in Erythrozyten vorhanden ist, oder mit CD42b-Antigen, das im Allgemeinen auf Thrombozyten vorhanden ist, oder mit CD45-Antigen, das im Allgemeinen auf Leukozyten vorhanden ist;
e) Durchführen eines immunchromatographischen Assays für jeden markierten Rezeptor, der einen der Zielanalyten Hämoglobin, CD42b und CD45 gebunden hat; und
f) Quantifizieren der Mengen jedes der Zielanalyten CD45, CD42b und Hämoglobin; und
g) Vergleichen und Korrelieren der Mengen an CD45, CD42b und Hämoglobin mit den erwarteten Werten und Bereichen, die bei gesunden Personen und/oder zuvor gemessenen Werten gefunden wurden, wobei Werte außerhalb des individuell akzeptablen Bereichs oder des normalen gesunden Bereichs darauf hinweisen, dass die untersuchte Person von einer Therapie profitieren wird.

9. Verfahren zur Durchführung einer Blutkörperchenzählung, umfassend die Quantifizierung von roten Blutkörperchen (Erythrozyten), weißen Blutkörperchen (Leukozyten) und Blutplättchen (Thrombozyten) in einer Vollblutprobe, die unter Verwendung eines immunologischen Assays gemäß einem der Ansprüche 1 bis 8 entnommen wurde, ferner umfassend die Verwendung eines Kits von Teilen zur Durchführung einer Blutkörperchenzählung gemäß einem der Ansprüche 1 bis 8, umfassend:
- eine Vorrichtung zum Entnehmen einer definierten Menge Vollblut durch Kapillarwirkung;
- ein Gefäß, das einen Zelllyse- und Extraktionspuffer zum Verdünnen der entnommenen definierten Blutmenge, zum Aufbrechen der Blutzellen, zur Proteinextraktion und zur Solubilisierung von Single-Pass-Transmembranproteinen enthält;
- ein oder mehrere spezifische immunchromatographischen Testsysteme für CD45-Antigen, CD42b-Antigen und Hämoglobin.

10. Verfahren zur Durchführung einer Blutzellzählung, umfassend die Quantifizierung von roten Blutkörperchen (Erythrozyten), weißen Blutkörperchen (Leukozyten) und Blutplättchen (Thrombozyten) in einer Vollblutprobe, die unter Verwendung eines immunologischen Assays gemäß einem der Ansprüche 1 bis 8 und eines Kits mit Teilen gemäß Anspruch 9 entnommen wurde, weiterhin umfassend die Verwendung einer Software zur Verwendung mit einem spezifischen immunchromatographischen Testsystem und Verfahren gemäß einem der Ansprüche 1 bis 9 und in einem Mobiltelefon, das eine Digitalkamera, eine Lichtquelle und einen Prozessor umfasst, wobei die Software die Aufnahme digitaler Bilder, das Lesen maschinenlesbarer Darstellungen von Daten, den Austausch von Daten und Bilddaten mit einem Remote-Server und die Darstellung von Informationen unterstützt, Daten und Testergebnisse auf einem Display, wobei die Software so konfiguriert ist, dass sie mehrere vorübergehende digitale Bilder nacheinander verarbeitet und jedes digitale Bild auf das Vorhandensein eines Referenzbildes analysiert und jedes vorübergehende digitale Bild auf einen interessierenden Bereich analysiert, der die spezifischen Ergebnisse der immunchromatographischen Testsysteme anzeigt, und, falls gefunden, so konfiguriert ist, dass sie jeden interessierenden Bereich auf die gemessenen Lichtreflexionen untersucht; und so konfiguriert ist, dass sie die Bilddaten des vorübergehenden digitalen Bildes für eine weitere Analyse der interessierenden Bereiche speichert, um durch Vergleich die Menge an Hämoglobin, die Anzahl der Leukozyten (weiße Blutkörperchen) und die Anzahl der Thrombozyten (Blutplättchen) in der entnommenen Vollblutprobe zu bestimmen.

## Revendications

1. Procédé de quantification des globules rouges (érythrocytes), des globules blancs (leucocytes) et des plaquettes (thrombocytes) dans un échantillon de sang total d'un individu, le procédé comprenant : -
a. fournir une quantité définie d'un échantillon de sang total prélevé sur ledit individu ;
b. la dilution et le traitement de la quantité définie de sang total dans un tampon de lyse cellulaire pour une lyse cellulaire efficace et la solubilisation des protéines de la membrane cellulaire ;
c. l'application de la solution obtenue contenant les protéines solubilisées et les particules provenant des cellules sanguines sur un tampon d'échantillonnage d'une bandelette de test immunochromatographique qui comprend une membrane ou un non-tissé imperméable aux particules et perméable aux protéines solubilisées ;
d. mettre en contact les protéines solubilisées de l'échantillon de sang total avec des récepteurs marqués se liant à l'hémoglobine généralement présente dans les érythrocytes, ou à l'antigène CD42b généralement présent sur les thrombocytes, ou à l'antigène CD45 généralement présent sur les leucocytes ;
e. réaliser un test immunochromatographique pour chaque récepteur marqué qui s'est lié à l'un des analytes cibles, à savoir l'hémoglobine, le CD42b et le CD45 ; et
f. quantifier les quantités de chaque analyte cible CD45, CD42b et hémoglobine ; et
g. comparer et corréler les niveaux de CD45, CD42b et d'hémoglobine avec les valeurs et les plages attendues chez des individus en bonne santé et/ou précédemment mesurées dans le sang d'un individu chez lequel une néoplasie a été diagnostiquée, les valeurs situées en dehors de la plage autorisée pour l'individu ou d'une plage normale indiquant que l'individu étudié tirera bénéfice du traitement.

2. Procédé selon la revendication 1, dans lequel une quantité définie de sang total est prélevée par capillarité et extraite à l'aide d'une quantité prédéterminée de tampon de lyse cellulaire comprenant des détergents pour l'extraction et la solubilisation des protéines transmembranaires à passage unique afin d'obtenir un extrait de cellules sanguines.

3. Procédé selon la revendication 2, dans lequel une quantité définie de fragment recombinant de CD42b et/ou CD45 ou de protéine membranaire à passage unique isolée est ajoutée à l'extrait de cellules sanguines pour permettre la quantification par immunoessai compétitif.

4. Procédé selon l'une quelconque des revendications 1 à 3, dans lequel l'extrait de cellules sanguines est appliqué en aliquotes égales sur des tampons conjugués séparés comprenant respectivement des anticorps anti-CD45 marqués, des anticorps anti-CD42b marqués et des anticorps anti-hémoglobine marqués.

5. Procédé selon l'une quelconque des revendications 1 à 3, dans lequel l'extrait de cellules sanguines est appliqué sur un tampon conjugué unique dans lequel le système de test immunochromatographique comporte des zones de liaison pour l'antigène CD45, l'antigène CD42 et l'hémoglobine.

6. Procédé selon l'une quelconque des revendications 1 à 5 précédentes, dans lequel un ou plusieurs tampons conjugués contiennent des quantités définies d'anticorps marqués contre une protéine sérique humaine abondante ou une protéine sanguine courante à titre de contrôle.

7. Procédé selon l'une quelconque des revendications 1 à 3, dans lequel des anticorps dirigés contre l'hémoglobine, l'antigène CD45, l'antigène CD42b et la protéine témoin sont contenus dans le tampon de lyse et d'extraction cellulaire ou ajoutés directement au tampon de lyse et d'extraction cellulaire.

8. Procédé de réalisation d'un hémogramme comprenant la quantification des globules rouges (érythrocytes), des globules blancs (leucocytes) et des plaquettes (thrombocytes) dans un échantillon de sang total prélevé à l'aide d'un test immunologique comprenant : -
a) la fourniture d'une quantité définie d'un échantillon de sang total prélevé sur un individu ou une personne sous chimiothérapie ou souffrant d'une tumeur solide ou hématologique ;
b) la dilution et le traitement de la quantité définie de sang total dans un tampon de lyse cellulaire pour une lyse cellulaire efficace et la solubilisation des protéines de la membrane cellulaire ;
c) l'application de la solution obtenue contenant les protéines solubilisées et les particules provenant des cellules sanguines sur un tampon d'échantillonnage d'une bandelette de test immunochromatographique comprenant une membrane, une toison ou un tissu non tissé imperméable aux particules et perméable aux protéines solubilisées ;
d) mettre en contact les protéines solubilisées de l'échantillon de sang total avec des récepteurs marqués se liant à l'hémoglobine généralement présente dans les érythrocytes, ou à l'antigène CD42b généralement présent sur les thrombocytes, ou à l'antigène CD45 généralement présent sur les leucocytes ;
e) réaliser un test immunochromatographique pour chaque récepteur marqué qui s'est lié à l'un des analytes cibles hémoglobine, CD42b et CD45 ; et
f) quantifier les quantités de chacun des analytes cibles CD45, CD42b et hémoglobine ; et
g) comparer et corréler les quantités de CD45, CD42b et hémoglobine aux valeurs et plages attendues chez les individus en bonne santé et/ou précédemment mesurées, les valeurs situées en dehors de la plage acceptable individuellement ou de la plage normale de santé indiquant que l'individu étudié tirera bénéfice du traitement.

9. Procédé de réalisation d'un comptage des cellules sanguines comprenant la quantification des globules rouges (érythrocytes), des globules blancs (leucocytes) et des plaquettes (thrombocytes) dans un échantillon de sang total prélevé à l'aide d'un test immunologique tel que revendiqué dans l'une quelconque des revendications 1 à 8, comprenant en outre l'utilisation d'un kit de pièces pour réaliser un comptage sanguin tel que revendiqué dans l'une quelconque des revendications 1 à 8, comprenant :
- un dispositif pour prélever une quantité définie de sang total par capillarité ;
- un récipient comprenant un tampon de lyse et d'extraction cellulaire pour la dilution de la quantité définie de sang prélevée, la lyse des cellules sanguines, l'extraction des protéines et la solubilisation des protéines transmembranaires à passage unique ;
- un ou plusieurs systèmes de test immunochromatographique spécifiques pour l'antigène CD45, l'antigène CD42b et l'hémoglobine.

10. Procédé de réalisation d'un comptage des cellules sanguines comprenant la quantification des globules rouges (érythrocytes), des globules blancs (leucocytes) et des plaquettes (thrombocytes) dans un échantillon de sang total prélevé à l'aide d'un test immunologique tel que revendiqué dans l'une quelconque des revendications 1 à 8 et d'un kit de pièces tel que décrit dans la revendication 9, comprenant en outre l'utilisation d'un logiciel destiné à être utilisé avec un système et un procédé de test immunochromatographique spécifiques selon l'une quelconque des revendications 1 à 9 et dans un téléphone mobile comprenant un appareil photo numérique, une source de lumière et un processeur, lequel logiciel prend en charge la prise d'images numériques, la lecture de représentations de données lisibles par machine, l'échange de données et de données d'images avec un serveur distant, et la représentation d'informations, des données et des résultats de test sur un écran, dans lequel le logiciel est configuré pour traiter séquentiellement plusieurs images numériques transitoires et analyser chaque image numérique pour détecter la présence d'une image de référence, et pour analyser chaque image numérique transitoire à la recherche d'une région d'intérêt affichant les résultats spécifiques des systèmes de test immunochromatographiques, et, si elle est trouvée, est configuré pour examiner chaque région d'intérêt afin de mesurer la lumière réfléchie ; et est configuré pour enregistrer les données d'image de ladite image numérique transitoire en vue d'une analyse plus approfondie des régions d'intérêt afin de déterminer par comparaison la quantité d'hémoglobine, le nombre de leucocytes (globules blancs) et le nombre de thrombocytes (plaquettes) dans l'échantillon de sang total prélevé.
